# EUROPÄISCHE PATENTSCHRIFT

(11) **EP 0 335 265 B1**
(45) Veröffentlichungstag und Bekanntmachung des Hinweises auf die Patenterteilung: **02.10.1996**
(21) Anmeldenummer: 89105230.0
(22) Anmeldetag: 23.03.1989
(51) Int. Cl.: C07H 15/256, A23L 1/221, A23L 1/236, A23L 2/60

(54) **Verfahren zur Herstellung eines natürlichen Süssungsmittels auf der Basis von Stevia rebaudiana**
Process for the preparation of a natural sweetening agent derived from stevia rebaudiana
Procédé de préparation d'un agent de sucrage naturel dérivé de la stevia rebaudiana

(30) Priorität: 29.03.1988 DE 3810681
(43) Veröffentlichungstag der Anmeldung: 04.10.1989
(73) Patentinhaber: Kienle, Udo, D-70597 Stuttgart (DE)
(72) Erfinder: Kienle, Udo, D-70597 Stuttgart (DE)
(74) Vertreter: Andrae, Steffen, Dr.

(56) Entgegenhaltungen:
- US-A- 4 082 858
- US-A- 4 361 697
- CHEMICAL ABSTRACTS, Band 95, Nr. 21, 23. November 1981, Seite 522, Zusammenfassung Nr. 185762c, Columbus, Ohio, US; & JP-A-81 109 568 (MARUZEN CHEMICAL CO., LTD) 31-08-1981
- PARFUMS, COSMETIQUES, AROMES, Nr. 55, Februar-März 1984, Seiten 47-54; L. PEYRON: "Le dioxyde de carbone liquide et supercritique"
- CHEMICA ABSTRACTS, Band 110, Nr. 25, 19. Juni 1989, Seite 536, Zusammenfassung, Nr. 230444s, Columbus, Ohio, US; & JP-A-63 177 764 (MITSUI PETROCHEMICAL INDUSTRIES LTD) 21-07-1988
- CHEMICAL ABSTRACTS, Band 107, Nr. 17, 26. Oktober 1987, Seite 447, Zusammenfassung Nr. 151504w, Columbus, Ohio, US; & JP-A-62 00 496 (MITSUI PETROCHEMICAL INDUSTRIES, LTD) 06-01-1987

## Beschreibung

Die Erfindung betrifft ein Verfahren zur Herstellung eines natürlichen Süßungsmittels auf der Basis von Pflanzenteilen von Stevia rebaudiana oder eines aus diesen Pflanzenteilen gewonnen Extrakts sowie seine Verwendung.

Wie einem Übersichtsartikel von Abraham I. Bakal und Lyn O'Brien Nabors "Stevioside" in: "Alternative Sweeteners", Seite 295 bis 307, Marcel Dekker Inc., New York 1986 und den dort zitierten Literaturstellen entnommen werden kann, ist die Pflanze Stevia rebaudiana in Südamerika beheimatet und dient dort als traditionelles Süßungsmittel für den Mate-Tee. Aufgrund der hohen Süßungskraft der Inhaltsstoffe dieser Pflanze wurden außer in ihren Ursprungs ländern insbesondere in Ostasien erfolgreiche Versuche zur Kultivierung dieser Pflanze und kommerziellen Nutzung daraus gewonnener Extrakte unternommen. Der süße Geschmack der Pflanzenteile und der daraus gewonnen Extrakte geht auf eine Reihe von chemischen Substanzen zurück, die alle der Klasse der Diterpenglycoside angehören. Die wichtigsten Einzelverbindungen sind dabei die Verbindungen Steviosid und Rebaudisid A, die die nachfolgend angegebenen Strukturformeln aufweisen.

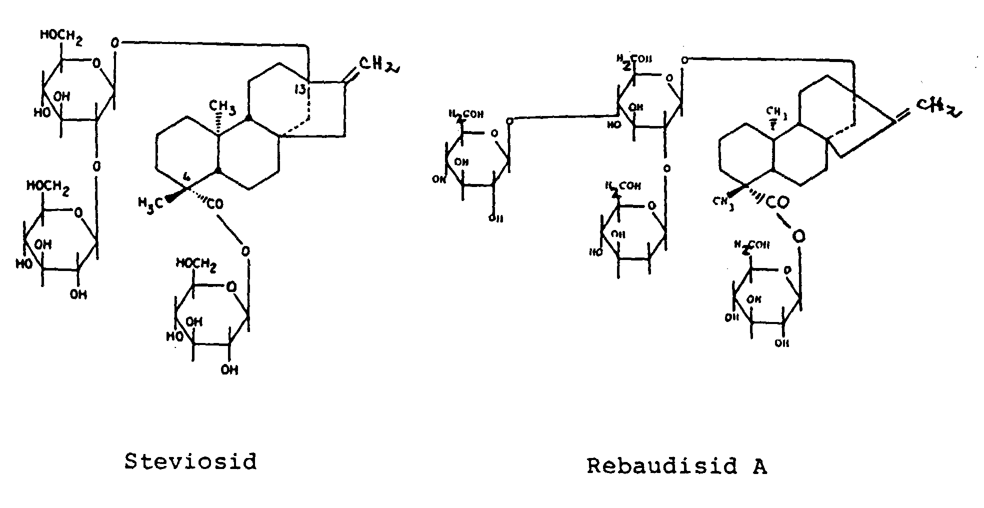

Außer diesen beiden Verbindungen wurden noch verschiedene weitere verwandte Verbindungen identifiziert, die einen Beitrag zur Süßkraft liefern.

Zur Gewinnung von Süßungsmitteln aus Stevia rebaudiana werden die Pflanzenteile, üblicherweise die getrockneten Blätter, mit Wasser oder organischen Lösungsmitteln wie Alkoholen extrahiert. Die Extrakte werden anschließend zur Reinigung und Anreicherung der für die Süßkraft entscheidenden Inhaltsstoffe verschiedenen Raffinationsprozessen unterzogen, die den folgenden Verfahrenstypen zugeordnet werden können: Ausfällung von Verunreinigungen mit Hilfe von anorganischen Salzen und anschließende Nachbehandlung des Extrakts mit Ionenaustauschern; Ausfällen der Verunreinigungen durch gezieltes Verändern des pH-Wertes; Ausfällen unerwünschter Begleitsubstanzen durch Aggregation an Polymere oder feste Adsorbenzien; Aufreinigung durch Chromatographieverfahren; Aufreinigung durch Adsorption von Pigmenten; Flüssigextraktion z.B. gemäß JP-Kokai Tokkyo Koho 81-109568; Elektrophoreseverfahren; Membranfiltration.

Die Anzahl der in Chemical Abstracts 85 bis 105 referierten Veröffentlichungen, die überwiegend veröffentlichte japanische Patentanmeldungen sind, liegt über 70. Aus den Rohextrakten erhaltene Produkte mit verbesserter Süßkraft sind beispielsweise auch in den US-PSen 4 082 858 und 4 219 571 beschrieben, wobei die letztgenannte Patentschrift ein Verfahren zur enzymatischen Herstellung betrifft.

Die Süßkraft des Extraktes, der gelegentlich einfach als "Steviosid" bezeichnet wird, wird im allgemeinen mit dem ca. 150 bis 300fachen von Saccharose angegeben, und zwar abhängig von der Einsatzkonzentration.

Der Einführung von Süßungsmitteln, die aus Stevia rebaudiana gewonnen wurden, steht in Europa und Nordamerika ein bitterer und adstringenter Nachgeschmack, der gelegentlich auch als mentholartig beschrieben wird, entgegen. Dieser Nachgeschmack bleibt bei allen bekannten, kommerziell anwendbaren Verfahren im Extrakt, und dieser Nachgeschmack bringt gerade bei hoher Reinheit des Extrakts oder einer hohen Einsatzkonzentration sehr starke geschmackliche Nachteile. Dieser Bei- oder Nachgeschmack wird in den Ländern, in denen Stevia-Süßstoff in Lebensmitteln und Getränken kommerziell eingesetzt wird, toleriert. Ein Produkt dieser Qualität aber würde von Konsumenten in Europa und in Nordamerika nicht akzeptiert werden. Darüber hinaus limitiert der bittere Nachgeschmack die Einsatzmöglichkeiten der bekannten Stevia-Süßstoffe dahingehend, daß sie zur Süßung von alkoholfreien Getränken (Softdrinks) nicht verwendet werden können.

Der vorliegenden Erfindung liegt die Aufgabe zugrunde, ein neues und für die kommerzielle Anwendung geeignetes Verfahren zur Herstellung eines natürlichen Süßungsmittels auf der Basis von Pflanzenteilen von Stevia rebaudiana oder eines aus diesen Pflanzenteilen gewonnenen Extrakts zu schaffen, bei dem ein derartiges Süßungsmittel ohne den seine Verwendung störenden Bei- oder Nachgeschmack bei voller Bewahrung der Süßkraft erhalten werden kann.

Diese Aufgabe wird bei einem Verfahren der genannten Art durch die im Kennzeichen des Patentanspruchs 1 wiedergegebenen Maßnahmen gelöst.

Vorteilhafte Ausgestaltungen des Verfahrens sind den Unteransprüchen zu entnehmen.

Die Beseitigung des störenden Beigeschmacks nach dem erfindungsgemäßen Verfahren ermöglicht die Verwendung der so erhaltenen Süßungsmittel für ein breites Spektrum von Lebensmitteln und Getränken, insbesondere auch für Softdrinks, für die die bisherigen Stevia-Extrakte ungeeignet waren. Keiner der zahlreichen Literaturstellen ist ein Hinweis darauf zu entnehmen, daß die geschmacklich störenden Pflanzeninhaltsstoffe, die sich nicht nur in den Blättern von Stevia rebaudiana finden, sondern in alle Extrakte und auch enzymatischen Umwandlungsprodukte aus Steviosid gelangten, durch Extraktion mit überkritischen Gasen, insbesondere mit überkritischem Kohlendioxid, oder mit flüssigem Kohlendioxid auf kommerziell vorteilhafte Weise entfernt werden können.

Die Extraktion mit überkritischen Gasen ist inzwischen zu den bekannten Extraktionsverfahren zu zählen. Eine Zusammenfassung des bisherigen Erfahrungswissens zu diesem Extraktionsverfahren sowie der bisherige Anwendungs- und Erprobungsbereich dieses Verfahrens können beispielsweise entnommen werden dem Buch von E. Stahl, K.W. Quirin, D. Gerard "Verdichtete Gase zur Extraktion und Raffination", Springer-Verlag, Berlin, Heidelberg, New York, London, Paris, Tokyo, 1987. Auf die Ausführungen in diesem Buch zur üblichen Durchführung von Extraktionsverfahren mit überkritischen Gasen, insbesondere mit überkritischem Kohlendioxid, wird ausdrücklich ergänzend Bezug genommen, da das erfindungsgemäße Verfahren, was seine technische Durchführung angeht, grundsätzlich bekannten Verfahren entspricht und somit in vielen üblichen Anlagen durchgeführt werden kann.

Aus der JP-A-62-000496 ist der Versuch bekanntgeworden, das Süßungsmittel Steviosid aus Blättern von Stevia rebaudiana mit Hilfe von überkritischem Kohlendioxid zu extrahieren. Es wird berichtet, daß bei Verwendung von überkritischem Kohlendioxid kein Steviosid extrahiert werden kann, daß bei einem Zusatz von 0,5 bis 50 Mol -% Methanol, Ethanol oder Aceton oder mit einem tertiären Gemisch aus überkritischem Kohlendioxid, Wasser und Methanol jedoch eine Steviosidextraktion möglich ist. Das bei der beschriebenen Extraktion erhaltene Steviosid ist stark mit zahlreichen unerwünschten Begleitstoffen und insbesondere mit allen den Geschmack beeinträchtigenden Begleitstoffen verunreinigt. Als Reinigungsverfahren, insbesondere als Reinigungsverfahren eines auf übliche Weise gewonnenen Steviosidextrakts, wird die Extraktion mit überkritischem Kohlendioxid nicht in Erwägung gezogen. Da die chemische Natur der für den unerwünschten Nachgeschmack verantwortlichen Inhaltsstoffe unzureichend bekannt ist, war eine einfache Übertragung von Kenntnissen des Standes der Technik auf die Gewinnung eines Steviosids ohne unerwünschten Beigeschmack nicht möglich.

Es war daher, auch gerade angesichts der grundsätzlichen Bekanntheit des Extraktionsverfahrens mit überkritischen Gasen, außerordentlich überraschend, als festgestellt wurde, daß bei einer Extraktion mit überkritischen Gasen eine weitgehende bis vollständige Entfernung der für den bitteren oder adstringenten Nachgeschmack verantwortlichen Inhaltsstoffe von Stevia rebaudiana möglich ist, und zwar nicht nur aus den frischen oder getrockneten Pflanzenteilen wie Blättern, sondern auch aus allen in den Handel gelangten Extrakten oder enzymatischen Umwandlungsprodukten.

Durch das erfindungsgemäße Verfahren werden die geschmacklichen Eigenschaften des Süßextrakts deutlich verbessert. Dadurch werden seine Einsatzmöglichkeiten dahingehend vergrößert, daß höhere Süßkonzentrationen erzielt werden können, wie z. B. für Soft-Drinks, ohne daß die Konsumentenakzeptanz abnimmt.

Im erfindungsgemäßen Verfahren wird als Extraktionsmittel zur Raffination von Stevia-Blättern oder Stevia-Extrakten der verschiedensten Art ein überkritisches Gas verwendet. Es wurde festgestellt, daß der gewünschte Effekt bei Verwendung einer Reihe von Gasen erhalten wird, wobei sich die verschiedenen Gase in ihrer Wirksamkeit durchaus voneinander unterscheiden. So werden die bitteren und adstringierenden Bestandteilen von Stevia rebaudiana von Ethan, Ethen, Distickstoffmonoxid, Propan und Propen besser gelöst als von Kohlendioxid oder verschiedenen Fluorwasserstoffen. Als überkritisches Gas kann auch Stickstoff verwendet werden (kritische Temperatur: - 147,10°C bzw. 126,05°K; kritischer Druck: 33,9 bar).

Aufgrund der mit der Verwendung organischer Extraktionsmittel verbundenen bekannten Nachteile und aufgrund der Tatsache, daß Distickstoffmonoxid als die Verbrennung unterhaltendes Gas nur mit Vorsicht gehandhabt werden kann, ist trotzdem Kohlendioxid für das erfindungsgemäße Verfahren das bevorzugte Gas. Die Erfindung wird daher nachfolgend insbesondere im Hinblick auf die Verwendung von überkritischem Kohlendioxid beschrieben.

Die erforderlichen Abwandlungen des erfindungsgemäßen Verfahrens, insbesondere die kritischen Temperaturen und Drücke sowie Dichten für die oben diskutierten weiteren Extraktionsmittel, sind dem Stand der Technik zu entnehmen (vgl. z. B. E. Stahl et al, a.a.O,Seite 15). Im Gegensatz zu den anderen genannten Gasen ist Kohlendioxid physiologisch unbedenklich, nicht brennbar oder explosiv, ist umweltfreundlich und steht in großen Mengen zu günstigen Bedingungen überall zur Verfügung. Ferner sind auch Extraktionsverfahren unter Verwendung von überkritischem Kohlendioxid gut ausgearbeitet. Kohlendioxid wird dabei mittels eines Druckerzeugers auf überkritische Bedingungen (Druck über 72,9 bar, Temperatur über 31°C) gebracht. Das überkritische Gas wird mittels eines Wärmetauschers isobar auf die Extraktionstemperatur gebracht. Dann wird das überkritische Kohlendioxid der gewünschten Temperatur in einen Extraktionsbehälter geleitet, in dem sich das zu behandelnde Rohmaterial befindet. Es ist ferner möglich, das überkritische Kohlendioxid auch bei kontinuierlicher Verfahrensführung durch ein Extraktionsgut zu leiten.

Nach der Extraktion wird das extraktbeladene Gas auf einen Druck unterhalb von 72,9 bar entspannt. Dabei kühlt sich das Gas ab und liegt als Naßdampf vor. Es bilden sich eine extraktreiche Flüssigphase und eine extraktreiche Gasphase. Zur Abscheidung wird das Gas verdampft und dann isobar auf die Abscheidungstemperatur gebracht. Dafür sind Temperaturen zwischen 25 bis 50°C sinnvoll.

Nach dem Abscheiden wird das regenerierte Gas auf Verflüssigungstemperatur gekühlt und dem Prozeß erneut zugeführt.

Das erfindungsgemäße Verfahren folgt diesem grundsätzlich bekannten Verfahrensschema. Die Extraktionstemperatur soll dabei, in Abhängigkeit von dem eingesetzten Ausgangsmaterial, zwischen 31°C und 100°C liegen. Zur effektiven Extraktion der geschmacklich störenden Begleitstoffe ist ferner ein richtiger Massendurchsatz des zu behandelnden Ausgangsmaterials und der Menge des Kohlendioxids zu beachten.

Es ist darauf hinzuweisen, daß die Extraktionstemperatur jedoch auch über 100°C liegen kann, und zwar dann, wenn ein Extrakt aus Stevia rebaudiana eingesetzt wird, der mit Hilfe von über 100°C siedenden organischen Lösungsmitteln gewonnen wurde.

Bei dem erfindungsgemäßen Verfahren kann dieser relative Massendurchsatz zwischen 5 kg CO₂/kg Ausgangsmaterial und bis zu 310 kg Kohlendioxid/kg Ausgangsmaterial variiert werden. Üblicherweise liegen die Mengenverhältnisse zwischen 8 kg Kohlendioxid/kg Ausgangsmaterial und 100 kg Kohlendioxid/Ausgangsmaterial. Bei einem Massenverhältnis unter 5kg Kohlendioxid/kg Ausgangsmaterial ist keine spürbare Entfernung der störenden Geschmacksstoffe zu bemerken.

Ein optimales Verhältnis des relativen Massenverhältnisses bei bevorzugten Bedingungen von 60°C/300 bar liegt zwischen 8 und 100 kg Kohlendioxid/kg Ausgangsmaterial.

Bei der Verwendung von flüssigem Kohlendioxid kann bei Temperaturen im Bereich von 0 bis 31°C, vorzugsweise im Bereich von 5 bis 31°C, sowie bei Drücken im Bereich von 40 bis 72,9 bar gearbeitet werden. Das Verfahren unter Verwendung von flüssigem Kohlendioxid als Extraktionsmittel ähnelt dabei weitgehend dem mit überkritischem Kohlendioxid, wobei naturgemäß allerdings der Schritt einer Entspannung des extraktbeladenen Extraktionsmittels auf einen unterkritischen Druck nicht erforderlich ist.

Wird das Ausgangsmaterial in einer der nachfolgend und in den Patentansprüche beschriebenen Einsatzformen mit Kohlendioxid auf die beschriebene Weise in dem an sich bekannten Extraktionsverfahren behandelt, dann wird ein Raffinat erhalten, das als Süßungsmittel in Soft-Drinks in geeigneter Konzentration ohne geschmackliche Beeinträchtigung eingesetzt werden kann. Man erhält dadurch ein Süßungsmittel, das in seiner Qualität bessere Süßungseigenschaften aufweist als bekannte synthetische Süßstoffe. In seinem Geschmacksprofil ähnelt es nahezu dem gewohnten Zuckergeschmack.

Nachfolgend wird das erfindungsgemäße Verfahren noch anhand seiner verschiedenen Ausgestaltungsmöglichkeiten und der dafür vorteilhaften Abwandlungen näher beschrieben.

1. Behandlung von Blättern von Stevia rebaudiana Bertoni

Bei dieser Verfahrensvariante werden Blätter von Stevia rebaudiana Bertoni frisch oder getrocknet, ganz oder zerkleinert, in einen Extraktionsbehälter eingebracht. Nach dem Verschließen des Extraktionsbehälters wird überkritisches Kohlendioxid durch den Behälter geleitet. Dabei werden Cuticularwachse, Chlorophyll, andere Pigmente und insbesondere die geschmacklich störenden Begleitsubstanzen entfernt. Die in dem überkritischen Kohlendioxid gelösten, aus den Blättern entfernten Substanzen werden im Behälter 2 abgeschieden. Dabei wird das Kohlendioxid regeneriert und kann nach Verdichtung erneut dem Prozeß zugeführt werden.

Nach Beendigung des etwa 8stündigen Extraktionsvorgangs wird der Behälter geöffnet, und die Pflanzenteile werden entnommen. Sie können direkt verwendet werden, werden jedoch üblicherweise anschließend in bekannter Weise wie frische oder getrocknete Blätter aufgearbeitet. Dabei werden die süßschmeckenden Diterpenglykoside ohne geschmackliche Verunreinigungen isoliert.

Aufgrund der großen Menge des zu behandelnden Materials ist diese Art der Verfahrensführung jedoch wenig ökonomisch, selbst wenn man zur Erhöhung der Schüttdichte die getrockneten oder gemahlenen Blätter vor der Extraktion zu Pellets preßt.

2. Behandlung von bekannten pulverförmigen Extrakten aus Blättern von Stevia rebaudiana Bertoni

Ein pulverförmiger Extrakt, der irgendeiner der handelsüblichen oder nach bekannten Gewinnungsmethoden hergestellten Extrakte sein kann, wird in einen Extraktionsbehälter eingebracht und auf an sich bekannte Weise mit überkritischem Kohlendioxid behandelt. Dabei kann dem Gas ein Schleppmittel zugesetzt werden, das so ausgewählt wird, daß es unter den Extraktionsbedingungen in den angewandten Mengen zu keiner Extraktion von Steviosid führt. Wasser, ein C₁-C₄-Alkanol oder ein geeigneter Kohlenwasserstoff oder ein Gemisch der genannten Lösungsmitteln ist bevorzugt, wobei insbesondere höhere Alkohole den besten Effekt zeigen.

Auch in diesem Falle gelingt es, die störenden Geschmacksstoffe aus dem als Extraktionsrückstand erhaltenen Produkt teilweise zu entfernen. Bei dieser Art der Behandlung ist jedoch der Grad der Entfernung der geschmacklichen Begleitsubstanzen geringer, und ohne Schleppmittel werden nur unzureichende Erfolge erzielt. Bei dieser Verfahrensführung ist ferner die thermische Belastung des Extrakts nachteilig. Eine Extraktionstemperatur von 50°C darf zur Verhinderung von Qualitätsbeeinflussungen nicht überschritten werden.

3. Behandlung von Extraktlösungen aus Blättern von Stevia rebaudiana Bertoni

Extraktlösungen, in denen ein Extrakt aus Blättern von Stevia rebaudiana Bertoni in Wasser oder einem geeigneten Alkohol oder einem anderen organischen Lösungsmittel oder Lösungsmittelgemisch vorliegt, werden bei dieser Art der Verfahrensführung kontinuierlich in einen Extraktionsbehälter eingepumpt. Der Extraktionsbehälter ist mit Füllkörpern oder einer geordneten Packung gefüllt, um die Kontaktfläche zwischen Flüssigkeit und Gas zu erhöhen. Der Gaszustrom und das Zudosieren des flüssigen Extrakts sollen dabei so aufeinander abgestimmt sein, daß der relative Massenfluß zwischen 8 bis 50 kg Kohlendioxid/kg Trockensubstanz in der Extraktlösung liegt. Extraktionsdruck und Extraktionstemperatur liegen im überkritischen Bereich, wobei geeignete bevorzugte Bedingungen 300 bar und 60°C darstellen.

Bei einer auf diese Weise durchgeführten Extraktion, die die bevorzugte Verfahrensvariante darstellt, können geschmacklich störende Begleitstoffe sowie ein Teil anderer Verunreinigungen entfernt werden.

Es ist bei dieser Art der Verfahrensführung nicht wichtig, welchen Reinheitsgrad der in Form einer Lösung eingesetzte Extrakt aufweist. Der Extrakt kann ein Rohextrakt sein, kann schon nach dem bekannten Verfahren teilweise oder vollständig aufgereinigt sein oder kann auch erst nach der erfindungsgemäßen Behandlung einer zusätzlichen Reinigung unterzogen werden.

Wenn Extraktlösungen verwendet werden, bei denen das Lösungsmittel ein Alkohol oder ein organisches Lösungsmittel ist, sollten diese wasserhaltig sein und einen Mindestgehalt an Wasser aufweisen, damit der gereinigte Süßstoff in einer eigenen wäßrigen Phase gelöst kontinuierlich abgezogen werden kann.

Wenn Extraktlösungen in alkoholischen oder anderen organischen Lösungsmitteln verwendet werden, kann das Verfahren auf ebenfalls an sich bekannte Weise so gestaltet werden, daß die Lösungsmittel zurückgewonnen werden können, so daß der Lösungsmittelbedarf gering gehalten werden kann.

Ist der Reinheitsgrad des in das erfindungsgemäße Verfahren eingesetzten Extraktes bereits ausreichend hoch (Süßstoffgehalt von mehr als 40 %), kann der behandelte Extrakt direkt in flüssiger Form Lebensmitteln oder Getränken zugegeben werden, eine entsprechende Eignung des Lösungsmittels selbstverständlich vorausgesetzt.

4. Reinigung von rohem Steviosid und anderen aus Stevia rebaudiana Bertoni isolierten süßschmeckenden Substanzen sowie von enzymatischen Umwandlungsprodukten aus rohem Steviosid

Aus Extrakten von Stevia rebaudiana kann auf bekannte Art und Weise (vgl. eingangs zitierte Literatur) Steviosid und Rebaudisid A als Reinsubstanz abgetrennt werden. Die Substanzen fallen zuerst kristallin als Rohsteviosid bzw. als Rohrebaudisid A mit einer Reihe von Verunreinigungen an, die sich auf den Kristalloberflächen anlagern und bewirken, daß selbst diese isolierten Substanzen noch die geschilderten geschmacklichen Nachteile aufweisen.

Zur Raffination nach dem erfindungsgemäßen Verfahren wird das Rohsteviosid oder das Rohrebaudisid A in bekannter Weise gelöst. Die erhaltene Lösung wird wie oben unter 3. kontinuierlich dem Extraktionsbehälter zugeführt. Geschmacklich störende Substanzen werden dabei, genau wie bei den vorausgehend beschriebenen Verfahrensweisen,entfernt. Das geschmacklich verbesserte aufgereinigte Steviosid bzw. Rebaudisid A kann dann Nahrungsmittels bzw. Getränken zugesetzt werden.

Auch enzymatisch in Rebaudisid A umgewandelte Lösungen von Rohsteviosid können nach dem erfindungemäßen Verfahren zur Gewinnung eines geschmacklich verbesserten natürlichen Süßungsmittels behandelt werden.

Für die Verwendung auf lebensmitteltechnologischem Gebiet oder durch den Endverbraucher kann das erfindungsgemäß gewonnene raffinierte Süßungsmittel in verschiedener, an sich bekannter Weise aufbereitet werden. Wie im Hinblick auf die Verfahrensführung unter Einsatz von Extraktlösungen beschrieben, können derartige gereinigte Extraktlösungen direkt in flüssiger Form einer Endverwendung zugeführt werden. Die gereinigten Extrakte oder das gereinigte Steviosid oder Rebaudisid A können auch wieder in feste kristalline Form überführt werden und in dieser Form an einen gewerblichen oder privaten Endverbraucher geliefert werden. Aufgrund der hohen Süßungskraft der Stevia-Extrakte werden diese noch mit einem geschmacklich neutralen Streckmittel verdünnt, wenn sie als Mittel zur Süßung von Getränken wie Tee oder Kaffee an den Endverbraucher geliefert werden sollen. Eine geeignete Vertriebsform sind kleine Papierbeutel mit einer z. B. einem Stück Würfelzucker entsprechenden Süßkraft, die einen Stevia-Extrakt in Verdünnung mit löslicher Stärke enthalten.

## Patentansprüche

1. Verfahren zur Herstellung eines natürlichen Süßungsmittels auf der Basis von Pflanzenteilen von Stevia rebaudiana oder eines aus diesen Pflanzenteilen gewonnenen Extrakts, dadurch gekennzeichnet, daß man zur Entfernung von für einen nichtsüßen Beigeschmack verantwortlichen Begleitsubstanzen
a) die Pflanzenteile, insbesondere die Blätter, zuerst einer Extraktion mit einem überkritischen Gas unterwirft und anschließend den Extraktionsrückstand zur Gewinnung des natürlichen Süßungsmittels einer an sich bekannten Extraktion mit einem wäßrigen oder organischen Lösungsmittel unterwirft und den Extrakt gewinnt, oder
b) einen durch an sich bekannte Extraktion mit einem wäßrigen oder organischen Lösungsmittel aus Stevia rebaudiana gewonnenen Extrakt, der ggf. einer an sich bekannten zusätzlichen enzymatischen Behandlung unterworfen worden sein kann, einer Extraktion mit einem überkritischen Gas unterwirft und den Extraktionsrückstand gewinnt.

2. Verfahren nach Anspruch 1, dadurch gekennzeichnet, daß das überkritische Gas Kohlendioxid, Stickstoff, Ethan, Ethen, Distickstoffmonoxid, Propan oder Propen ist.

3. Verfahren nach Anspruch 2, dadurch gekennzeichnet, daß das überkritische Gas Kohlendioxid ist und bei einer Temperatur von über 31°C, insbesondere im Bereich von 31 bis 100°C, bei einem Druck über 72,9 bar und in einer Menge, bezogen auf die Trockensubstanz des der Extraktion unterworfenen Ausgangsmaterials, von 5 bis 310 kg CO₂/kg Trockensubstanz eingesetzt wird.

4. Verfahren nach Anspruch 3, dadurch gekennzeichnet, daß eine Temperatur im Bereich von 50 bis 70°C und ein Druck zwischen 100 und 400 bar, insbesondere zwischen 250 bis 350 bar, angewandt werden, und daß das überkritische Kohlendioxid in einer Menge von 8 bis 310 kg CO₂/kg Trockensubstanz eingesetzt wird.

5. Verfahren nach einem der Ansprüche 1 bis 4, dadurch gekennzeichnet, daß als Ausgangsmaterial grüne oder getrocknete Blätter von Stevia rebaudiana, ggf. in zerkleinerter Form, eingesetzt werden, und die als Extraktionsrückstand gewonnenen Blätter direkt gewonnen oder einer weiteren, an sich bekannten Extraktion mit einem wäßrigen oder organischen Lösungsmittel zur Gewinnung eines Extrakts unterzogen werden.

6. Verfahren nach einem der Ansprüche 1 bis 4, dadurch gekennzeichnet, daß als Ausgangsmaterial eine Lösung eines Extrakts in einem wäßrigen oder organischen Lösungsmittel eingesetzt wird.

7. Verfahren nach Anspruch 6, dadurch gekennzeichnet, daß man die Extraktlösung in einem mit Füllkörpern gefüllten Extraktionsbehälter mit dem überkritischen Gas extrahiert, wobei der Zustrom des überkritischen Gases und die zugeführte Menge des Flüssigextraktes so aufeinander abgestimmt sind, daß der relative Massenfluß zwischen 5 bis 50 kg CO₂/kg Trockensubstanz, vorzugsweise zwischen 8 bis 50 kg CO₂/kg Trockensubstanz, liegt.

8. Verfahren nach Anspruch 7, dadurch gekennzeichnet, daß die der Extraktion unterworfene Extraktlösung wasserhaltig ist.

9. Verfahren nach einem der Ansprüche 1 bis 4, dadurch gekennzeichnet, daß ein fester Extrakt in Pulverform eingesetzt wird und daß die Extraktionstemperatur unter 50°C gehalten wird.

10. Verfahren nach einem der Ansprüche 1 bis 9, dadurch gekennzeichnet, daß die Extraktion in Gegenwart eines Schleppmittels durchgeführt wird.

## Claims

1. Process for the fabrication of a natural sweetener on the basis of the parts of the plant Stevia rebaudiana or on the basis of an extract obtained from these plant parts, characterized in that for the removal of the accompanying substances responsible for a non-sweet taste
a) the plant parts, especially the leaves, are first submitted to an extraction with a supercritical gas, and afterwards the extract residue is submitted to an in itself already known extraction with an aqueous or organic solvent for the production of the natural sweetener, and the extract is recovered, or
b) an extract, obtained through an in itself already known extraction with an aqueous or organic solvent from Stevia rebaudiana, which optionally has been submitted to an additional in itself already known enzymatic treatment, is submitted to an extraction with a supercritical gas, and the extract residue is recovered.

2. Process according to claim 1, characterized in that the supercritical gas is carbon dioxide, nitrogen, ethane, ethene, di-nitrogen-monoxide, propane or propene.

3. Process according to claim 2, characterized in that the supercritical gas is carbon dioxide which is used at a temperature of above 31°C, especially in the range from 31°C to 100°C, at a pressure of above 72,9 bar, and in an amount, based on the dry substance of the material which is submitted to the extraction, from 5 to 310 kg carbon dioxide/kg dry substance.

4. Process according to claim 3, characterized in that there are applied a temperature in the range from 50° to 70°C and a pressure between 100 and 400 bar, especially between 250 and 350 bar, and that the supercritical carbon dioxide is used in an amount from 8 to 310 kg CO₂/kg dry substance.

5. Process according to one of the claims 1 to 4, characterized in that green or dried leaves of Stevia rebaudiana are used as raw material, optionally in a crushed form, and that the leaves obtained as extraction residue are recovered directly or are submitted to another extraction with an aqueous or organic solvent which is in itself already known, to produce an extract.

6. Process according to one of the claims 1 to 4, characterized in that as raw material a solution of an extract in an aqueous or organic solvent is used.

7. Process according to claim 6, characterized in that the solution of the extract is extracted in a container filled with packings with a supercritical gas, the supply of the supercritical gas and the supplied amount of the liquid extract being coordinated such that the relative mass stream is between 5 to 50 kg CO₂/kg dry substance, preferably between 8 and 50 kg CO₂/kg dry substance.

8. Process according to claim 7, characterized in that the solution submitted to the extraction is aqueous.

9. Process according to one of the claims 1 to 4, characterized in that a solid extract in powder form is used and that the extraction temperature is below 50°C.

10. Process according to one of the claims 1 to 9, characterized in that the extraction is conducted in the presence of an extraction carrier.

## Revendications

1. Procédé pour la fabrication d'un édulcorant naturel à partir des parties végétales de stévia rebaudiana ou d'un extrait gagné de ces parties de la plante. Cet extrait est caractérisé par le fait que pour l'élimination des substances accompagnantes qui sont responsables pour un gout non-douc
a) on soumet les parties végétales, notamment les feuilles, à une extraction avec gaz super critique, ensuite le residu de l'extraction est soumis pour l'exploitation d'édulcorant naturel d'une extraction déjà connue en tant que telle avec un solvant aqueux ou organique et on gagne l'extrait ou,
b) on soumet un extrait, gagné d'une extraction déjà connue si necessaire avec un solvant d'eau ou organique, qui peut être soumit à d'un traitement enzymatique additionnel déjà connue en tant que tel, à une extraction avec un gas supercritique et gagne le residu d'extraction.

2. Le procédé d'après la spécification 1, est caractérisé de la manière que le gaz supercritique soit du gaz carbonique, d'azote, d'ethane, d'ethene, du monoxide diazote, du propane ou du propene.

3. Le procédé après la spécification 2, est caractérisé de la manière, que le gaz supercritique soit le gaz carbonique et qu'on emploie le gaz à une température de 31° à 100° C, à une pression au dessus de 72,9 bar et dans une quantité de 5 à 310 kg CO2/kg de substance sèche par rapport à la substance sèche de la matière de base.

4. Le procédé d'après la spécification 3, est caractérisé de la manière qu'une température entre environ 50 - 70°C et une pression entre 100 et 400 bar, notamment entre 250 jusq' au 350 bar soient utilisées, et que le gaz carbonique supercritque soit utilisé dans une quantité de 8 à 310 kg CO2 substance sèche.

5. Le procédé d'après une des spécifications 1 à 4, est caractérisé de la manière, qu'on utilise comme matière de base des feuilles vertes ou sèches et au besoin en forme broyées et les feuilles gagnées comme résiduelles d'extraction sont gagnées directement ou les feuilles sont soumit à une extraction déjà connue comme elle avec un solvant d'eau ou organique pour l'exploitation d'un extrait.

6. Le procédé d'après une des spécifiactions 1 à 4 , caractérisé de la manière, qu'on utilise une solution d'un extrait dans un solvant aqueux ou organique comme matière de base.

7. Le procédé d'après la spécification 6, est caractérisé de la manière, qu'on extrait la solution de l'extrait dans un contenant, rempli avec des corps de remplissage et avec le gaz supercritique; l'afflux du gas supercritique et la quantité alimentée de l'extraits liquides sont accordés de cette façon que le cours de masse relative se situe entre 5 à 50 kg CO2/kg substance sèche, de préférence entre 8 à 50 kg CO2/kg substance sèche.

8. Le procédé d'après la specification 7, est caractérisé de la manière, que la solution de l'extraction soumis à l'extraction soit aqueuse.

9. Le procédé d'après d'une des spécifications 1 à 4, est caractérisée de la manière , qu'on utilise un extrait solide pulvérulent et que la température de l'extraction soit maintenue en dessous de 50° C.

10. Le procédé d'après d'une des spécifications 1 à 9, est caracterisé de la manière, que l'extraction soit réalisée en présence d'un moyen de remorquage.
